# EUROPÄISCHE PATENTSCHRIFT

(11) **EP 1 905 355 B1**
(45) Veröffentlichungstag und Bekanntmachung des Hinweises auf die Patenterteilung: **21.09.2011**
(21) Anmeldenummer: 06019755.5
(22) Anmeldetag: 21.09.2006
(51) Int. Cl.: A61B 5/103, A61B 19/00, A61B 17/17, G03B 42/04, A61B 6/12, A61B 5/107

(54) **Beckenregistrierungsvorrichtung für die medizintechnische Navigation**
Hip registration system for medical navigation
Système de recalage de hanche pour navigation médicale

(43) Veröffentlichungstag der Anmeldung: 02.04.2008
(73) Patentinhaber: BrainLAB AG, 85622 Feldkirchen (DE)
(72) Erfinder: Henning, Stefan, 85570 Markt Schwaben (DE)
(74) Vertreter: Schwabe - Sandmair - Marx

(56) Entgegenhaltungen:
- EP-A- 1 632 193
- US-A- 4 341 220
- US-A- 5 681 326
- US-A- 6 143 003
- US-A1- 2004 102 792
- US-B1- 6 499 488

## Beschreibung

Die Erfindung betrifft eine Vorrichtung zur Registrierung der Position bzw. Ausrichtung eines Patientenbeckens für die medizintechnische Navigation.

Zur Vorbereitung eines chirurgischen Eingriffs im Bereich des Beckens muss in den Fällen, wo für den Eingriff eine Navigationsunterstützung (bildunterstützte Chirurgie) bereitgestellt werden soll, die räumliche Lage des Beckens ermittelt werden. Insbesondere ist hierbei von Interesse, dass ermittelt wird, wie spezielle anatomische Beckenebenen in dem Raum liegen, der durch das Koordinatensystem eines medizintechnischen Trackingsystems festgelegt wird, welches einem medizintechnischen Navigationssystem beigeordnet ist. Bei den wichtigen Ebenen handelt es sich beispielsweise um die vordere Beckenebene, die durch die beiden Darmbeinstachel und die beiden Schambeinstachel definiert wird, oder um die mittlere sagittale Beckenebene.

Die EP 1 632 193 A1 beschreibt ein Hüftregistrierungssystem mit einem Registrierungsrahmen, der mit reflektiven Markern versehen ist. Bei der Verwendung eines solchen Registrierungsrahmens muss gewährleistet sein, dass immer eine ausreichende Anzahl von Markern in freier Sichtlinie von den Trackingkameras des Trackingsystems erfasst werden können. Aus der US 2003/0153829 A1 ist ein Navigationssystem für Beckenoperationen bekannt, bei dem ein Patientenpositionierungsrahmen verwendet wird, an dem eine Referenzanordnung angebracht ist, die wiederum mit einem Pointer-Instrument registriert werden kann.

Es ist die Aufgabe der vorliegenden Erfindung, eine optimierte Registrierungsvorrichtung für ein Patientenbecken bereitzustellen. Insbesondere soll eine Registrierung mit der erfindungsgemäßen Vorrichtung in einem einzigen Schritt durchgeführt werden können.

Diese Aufgabe wird erfindungsgemäß durch eine Vorrichtung gemäß dem Anspruch 1 gelöst.

Eine erfindungsgemäße Vorrichtung zur Registrierung der Position bzw.- Ausrichtung eines Patientenbeckens für die medizintechnische Navigation umfasst einen Becken-Registrierungsrahmen, der Positionierungselemente trägt, die an definierten Beckenpunkten ansetzbar sind. Der Vorrichtung, insbesondere dem Rahmen, ist mindestens ein Positionsgeber zugeordnet, der in eindeutig definierbarer Position und/oder Ausrichtung gegenüber dem Rahmen angeordnet ist oder angeordnet werden kann.

Bei einer bevorzugten Ausführungsform der Erfindung ist die räumliche Ausrichtung des Rahmens bzw. die definierte Ausrichtung des Positionsgebers (oder mehrerer Positionsgeber) einer oder mehreren anatomischen Beckenebenen zugeordnet, also beispielsweise der mittleren Sagittalebene oder der vorderen Beckenebene. Vorzugsweise liegt der Positionsgeber bzw. liegen die Positionsgeber schon in einer solchen, speziell parallelen Ebene, bzw. sind in ihrer Ausrichtung zu einer parallelen Ebene definiert.

Der Positionsgeber ist ein elektromagnetischer Sensor, hier eine Magnettrackingspule, deren Position und/oder Ausrichtung von einem medizintechnischen Magnettrackingsystem erfassbar ist. Dabei ist es möglich, mehrere elektromagnetische Sensoren bzw. Magnettrackingspulen vorzusehen bzw. zusammen mit der Vorrichtung bereitzustellen. Der elektromagnetische Sensor oder die Sensoren bzw. die Magnettrackingspule oder die Spulen sind in fester Ausrichtung an dem Rahmen angebracht.

Es ist zu beachten, dass der Begriff "Positionsgeber" nicht zwangsläufig einen Sensor oder einen Marker bezeichnen muss; es könnte sich dabei theoretisch auch um den Magnetfelderzeuger handeln. Es ist nämlich denkbar, dass der Magnetfelderzeuger in definierter Anordnung an dem Patientenpositionierer angebracht ist, während der Sensor im Beckenknochen befestigt ist.

Der Rahmen weist mindestens eine Positionierungs- und/oder Ausrichtungshilfe, insbesondere eine Aufnahme, für einen handhabbaren Sensorträger auf, der einen elektromagnetischen Sensor bzw. eine Magnettrackingspule trägt. Die Ausrichtungshilfe bzw. die Aufnahme dient dann dazu, den Sensorträger richtungs- und/oder positionsbestimmend anzuordnen. Mit anderen Worten kann der Sensorträger mittels der Ausrichtungshilfe nur in einer bestimmten und vordefinierten Position oder Ausrichtung zum Rahmen mit Hilfe der Ausrichtungshilfe positioniert werden, so dass sich aus der Position des Sensorträgers bzw. des darin enthaltenen Sensors (Spule) auch die Ausrichtung des Rahmens bestimmen lässt.

Gemäß einem weiteren Aspekt der vorliegenden Erfindung können - entweder in Kombination mit den obigen Ausführungen oder allein stehend - mehrere Positionsgeber als Röntgenmarker ausgebildet sein, die aus einem für Röntgenstrahlung undurchlässigen Material bestehen und an dem Rahmen angeordnet sind, wobei der Rahmen zumindest in der Umgebung der Marker im Wesentlichen aus einem für Röntgenstrahlung durchlässigen Material besteht. Damit lässt sich der Registrierungsrahmen bzw. dessen Röntgenmarker in einem Röntgenbild (Fluoroskopiebild) abbilden, und aus den abgebildeten Markerpositionen lässt sich ebenfalls auf die räumliche Lage des Registrierungsrahmens und damit auch auf die Lage des Beckens zurückschließen.

Die Röntgenmarker können als Aufsätze oder als Einsätze an dem Rahmen angeordnet sein. Ferner besteht die Möglichkeit, den Rahmen und die Röntgenmarker so auszubilden, dass die Röntgenmarker einen Teil der Rahmenkontur bilden oder ersetzen, insbesondere einen Kanten- oder Eckenabschnitt.

Wenn hierin von einem röntgendurchlässigen Rahmen für die Fluoroskopie-Registrierung gesprochen wird, bedeutet dies nicht, dass es sich hierbei um die einzig mögliche Registrierungsmethode handelt. Es ist beispielsweise auch denkbar einen Patienten bzw. den Rahmen durch ein prä- oder intra-operativen CT-Scanner zu erfassen (wobei auch Röntgenstrahlen zum Einsatz kommen). Der Rahmen wäre bei der Verwendung der Fluoroskopie in zweidimensionalen "Fluoro-Bildern" zu erkennen, beim CT in zweidimensionalen Schichtbildern bzw. einem dreidimensionalen Datensatz. Die CT-basierte Registrierung kann also grundsätzlich auch immer dann zum Einsatz kommen, wenn hierin eine Fluoroskopie-Registrierung erwähnt wird.

Die Erfindung beschreibt also eine Beckenregistrierungsvorrichtung, die zum Registrieren der Beckenebenen und insbesondere, aber nicht zwingend, auch einer oder mehrerer anatomischer Landmarken (Darmbeinstachel=ASIS-Punkt=Anterior Superior Iliac Spine-Punkt; Schambeinstachel =Pubispunkt) geeignet ist Natürlich umfasst die Erfindung grundsätzlich auch diejenigen Verfahrensschritte bzw. Verfahren, die bei der Registrierung mit der erfindungsgemäßen Vorrichtung durchgeführt werden. Die Registrierung kann in einem Schritt durchgeführt werden, speziell auch für Patienten in seitlicher Liegeposition. Anders als bei bisherigen Verfahren und Systemen, beispielsweise bei denen gemäß der EP 1 632 193 A1, muss der Patient bei der Verwendung der erfindungsgemäßen Vorrichtung nicht mehr in Rückenlage registriert und danach in eine seitliche Position gebracht werden. Dieser Aufwand und eventuelle Sterilitätsprobleme lassen sich insbesondere mit den erfindungsgemäßen Vorrichtungen lösen, weil diese nicht mehr auf einer Trackingtechnologie basieren, welche von einer stehenden Sichtlinie zwischen Trackingkameras und optischen Markern abhängig ist. Des Weiteren kann durch elektromagnetische- oder Fluoro- oder CT-basierte Registrierung im Vergleich zur herkömmlichen Fluoro- oder Marker-Registrierung Zeit gespart werden, da nur ein Schritt bzw. weniger Fluoroskopiebilder nötig sind.

Da als Positionsgeber elektromagnetische Sensoren, hier Magnettrackingspulen verwendet werden, können diese in unterschiedlicher Weise an der mechanischen Rahmenvorrichtung angeordnet werden, um die Ausrichtung der Beckenebenen zu bestimmen. Der Vorteil liegt, wie gesagt, insbesondere darin, dass die Magnetsensoren nicht dauernd für ein Kamerasystem sichtbar sein müssen und die erforderlichen Ebenen- und Richtungsinformationen für jede vorstellbare Patientenposition messen können. Dies ist insbesondere dann von Vorteil, wenn schon Navigationsverfahren verwendet werden, die lediglich auf magnetischem Tracking basieren.

Ein einziger magnetischer Sensor reicht grundsätzlich aus, um die Richtung der Beckenebenen zu bestimmen und aufzuzeichnen. Abhängig von der Position der Ebenen und einem geeigneten Aufbau des Registrierungsrahmens bzw. einer geeigneten Anordnung des Magnetsensors könnte auch direkt die Position einer Ebene durch die Position des Sensors bestimmt werden. Hierbei ist lediglich dafür zu sorgen, dass der Sensor und seine Anordnung im Rahmen schon einer solchen Beckenebenenausrichtung entspricht.

Einerseits können die elektromagnetischen Sensoren bzw. Magnettrackingspulen, die fünfdimensionale bzw. sechsdimensionale Positions- und Richtungsinformationen liefern, starr bzw. fest am Rahmen angeordnet sein. Bei einer anderen Ausführungsform wird ein elektromagnetischer Sensor auf einem Träger vorgesehen, der beispielsweise die Form eines kleinen Stiftes (Pin) haben und mittels einer Ausrichtungshilfe in eindeutiger Position gegenüber dem Rahmen angeordnet werden kann.

Zusätzlich oder alternativ kann die Position der Beckenebenen unter Verwendung eines C-Bogen-Fluoroskopiegerätes registriert werden. Der Registrierungsrahmen wäre dann röntgenstrahlungsdurchlässig und würde implementierte Metallmarker oder Metallmarkerkanten aufweisen. Wenn hier oder hierin von Metallmarkern gesprochen wird, betrifft dies nur eine Ausführungsform. Natürlich kann es sich auch um ein anderes Material handeln, das Röntgenstrahlung absorbiert bzw. nicht durchlässt.

Die Software könnte automatisch die Position der Marker des jeweiligen Rahmens bzw. der als Marker ausgestalteten Ecken oder Kanten erfassen und auf die Ebenenposition zurückrechnen. Die registrierten Beckenebenen würden in Relation zu einer Beckenreferenzanordnung, einem elektromagnetischem Sensor am Becken oder einem elektromagnetischem Sensor am Rahmen positionell abgespeichert. Es würden keine zusätzlichen Fluoroskopieaufnahmen der ASIS- oder Pubispunkte notwendig werden, um die Beckenebenen zu registrieren. Der Patient könnte in seitlicher Position positioniert werden, während die Fluoroskopieaufnahmen erstellt werden. Ein weiterer Vorteil liegt in der verbesserten Sichtbarkeit der Rahmenmarker oder der Eckenmarker eines Beckenregistrierungsrahmens gegenüber den anatomischen Beckenlandmarken. Eine automatische Erfassung der Marker/Ecken in einem Fluoroskopiebild würde mit höherer Zuverlässigkeit funktionieren, da die Marker im Rahmen auf dem Bild deutlicher erkennbar sind (deutlicherer Kontrast) als z. B. Knochenteile, die von Weichteilgewebe ummantelt sind bzw. keine klare Knochenkanten aufweisen.

Die Erfindung wird im Weiteren anhand von Ausführungsformen und unter Bezugnahme auf die beiliegenden Zeichnungen näher erläutert. Sie kann alle hierin beschriebenen Merkmale einzeln sowie in jedweder sinnvollen Kombination umfassen. In den Zeichnungen zeigen:
- Figur 1: einen Beckenregistrierungsrahmen gemäß der vorliegenden Erfindung, wobei schematisch aufgezeigt ist, wie der Rahmen am Becken positioniert wird;
- Figur 2: einen Pin mit einem elektromagnetischen Sensor (Magnettrackingspule), eingebracht in eine Ausrichtungshilfe und in Relation zu einer Beckenebene dargestellt;
- Figur 3: eine schematische Darstellung eines Registrierungsrahmens mit Metallmarkern für die Fluoroskopie-Registrierung; und
- Figur 4: eine schematische, ausschnittsweise Darstellung einer Rahmenecke mit einem Eckmarker.

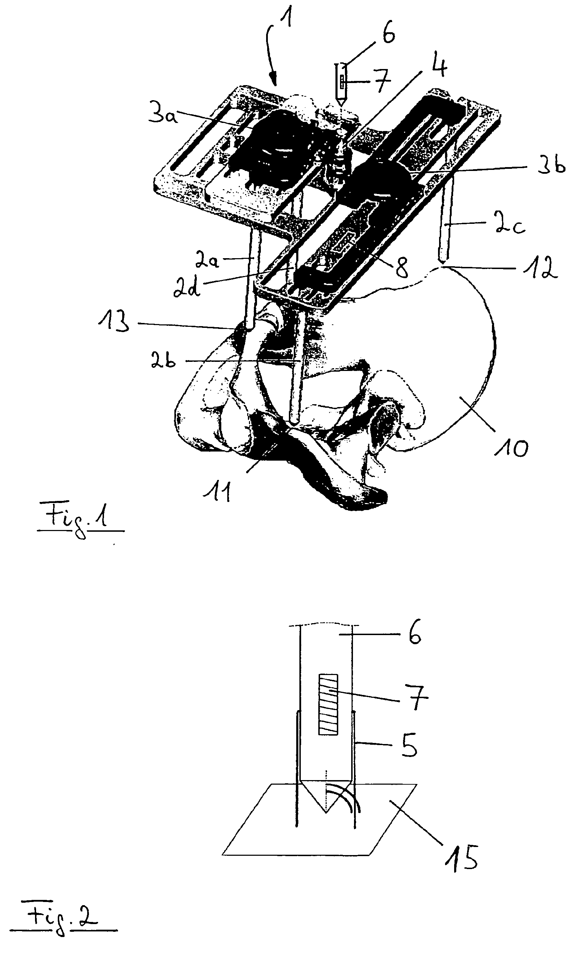

In der Figur 1 ist eine Becken-Registrierungsvorrichtung dargestellt, die einen Rahmen 1 aufweist, von dem stabförmige Positionierungselemente abstehen, welche die Bezugszeichen 2a, 2b, 2c und 2d tragen. Verstelleinrichtungen 3a und 3b gestatten es, die Positionierungselemente 2a und 2d bzw. 2b und 2c aufeinander zu bzw. voneinander weg zu fahren, um die Vorrichtung an eine patientenspezifische Anatomie anzupassen. Die Ebene, die von den Spitzen der Positionierungselemente bzw. von dem Rahmen 1 aufgebaut wird, ändert sich nicht durch die Breitenverstellung mit den Verstellelementen 3a und 3b.

Mit Hilfe des ebenfalls in Figur 1 dargestellten knöchernen Beckens 10 kann gezeigt werden, wie der Registrierungsrahmen 1 zur Registrierung der Beckenebenen auf spezielle Landmarken aufgesetzt wird, hier die beiden vorderen Darmbeinstachel 11 und 12, auf denen die Spitzen der Positionierungselemente 2b und 2c zu liegen kommen, sowie die beiden Schambeinstachel, von denen nur einer sichtbar und mit 13 bezeichnet ist. Auf den beiden Schambeinstacheln kommen die Spitzen der Positionierungselemente 2a und 2d zu liegen. Wenn der Registrierungsrahmen 1 derart am knöchernen Becken 10 angesetzt ist, steht die Lage seiner Elemente in einer definierten Beziehung zu der räumlichen Beckenposition und insbesondere zu den anatomischen Beckenebenen. Im vorliegenden Beispiel liegt der flache Teil des Rahmens 1 parallel zu einer Ebene, die als vordere Beckenebene bezeichnet wird, und die Ebene, die senkrecht dazu und jeweils auf dem halben Abstand zwischen den Elementen 2b und 2c sowie 2a und 2d liegt, ist die mittlere Sagittalebene des Beckens 10. In der Figur 1 ist noch schematisch ein elektromagnetischer Sensor 8 dargestellt, der eine Magnettrackingspule ist, deren Ausrichtung und Position (sechsdimensional bzw. in sechs Freiheitsgraden) innerhalb eines Magnettrackingsystems bestimmt werden kann, das noch einen hier nicht dargestellten Magnetfeldgenerator aufweist.

Ferner zeigt die Figur 1 noch, wie eine alternative oder zusätzliche Ausführungsform für einen Magnettracking-Positionsgeber aussehen kann, der als Pin 6 mit elektromagnetischem Sensor 7 (Magnettrackingspule 7) ausgestaltet ist. Der Pin 6 ist über einem stabartigen Fortsatz 4 gezeigt, der im vorliegenden Fall genau senkrecht auf der Ebene steht, die durch die Oberfläche des Rahmens 1 definiert wird.

In dem Fall, wo der starr am oder im Rahmen 1 angebrachte elektromagnetische Sensor 8 verwendet wird, muss dessen Positionierung gegenüber der oberen Rahmenebene bekannt sein, und dann kann man unmittelbar aus der vom Sensor 8 erhaltenen Richtungsinformation auf die Ausrichtung der Rahmenebene und damit auch auf die Ausrichtung der vorderen Beckenebene schließen. In den Fällen, wo zur Navigationsunterstützung lediglich die Lage dieser Ebenen bekannt sein muss, genügt demnach also schon die Ortung eines solchen mit dem Rahmen verbundenen Magnetsensors 8 und die Kenntnis der Rahmengeometrie.

Eine weitere Möglichkeit besteht darin, einen elektromagnetischen Sensor 7 an einem Träger anzusetzen, der im Weiteren als Pin 6 bezeichnet wird. Dieser Pin 6 kann in definierter Position gegenüber dem Rahmen an diesem angebracht oder in diesen eingesetzt werden. Im vorliegenden Fall geschieht dies beispielsweise über den senkrecht zur Rahmenebene stehenden stabartigen Fortsatz 4, und in schematischer Weise ist eine Positionierungsmöglichkeit in der Figur 2 dargestellt. Dort wird der Pin 6 mit dem darin ortsdefiniert angebrachten, hier axial angeordneten elektromagnetischen Sensor 7 in einen hohlen Abschnitt 5 des Fortsatzes 4 eingebracht. Damit ist die Ausrichtung des Pins 6 und auch des Sensors 7 definiert; sie steht genau senkrecht auf der Rahmenebene, die in der Figur 2 mit 15 bezeichnet ist. Wenn die Konstruktion vorteilhaft ausgeführt wird, ist diese Rahmenebene 15 auch gleichzeitig parallel zur vorderen Beckenebene. Aus der senkrechten Beziehung zwischen Magnetsensor 7 und der Beckenebene 15 lässt sich deren Ausrichtung bestimmen, ihre genaue Lage kann dann noch aus den Längen der Positionierungselemente 2a bis 2d, aus der Höhe des Rahmens sowie des Fortsatzes 4 und aus dem Abstand zwischen dem Sensor 7 und der Spitze des Pins 6 berechnet werden.

Wenn der Rahmen auf den vier anatomischen Punkten aufliegt, wird bei der bevorzugten Ausführungsform die Rahmenebene parallel zur Beckenebene liegen. Der Sensor könnte in einer definierten Richtung zu dieser Ebene liegen (d.h. der Software bekannt), muss aber nicht zwangsläufig senkrecht angebracht werden. Man könnte sich aber auch vorstellen, dass der Rahmen in seiner Ausführung nicht parallel zur Beckenebene gestaltet sein muss, solange die tatsächliche Ausrichtung vorher definiert wird und der verwendeten Software bekannt ist (durch Kalibrieren oder besser durch vordefinierte Programmierung). Das heißt, der Pin oder die Pins könnten aus ergonomischen Gründen schräg zum Rahmen angebracht sein.

Der Sensor 7 würde also in solcher Weise kalibriert, dass die Position und Ausrichtung des Sensorkoordinatensystems in einer festen Relation zu dem Pin 6 steht. Somit könnte der Pin 6 auch als Pointer-Vorrichtung angesehen werden, deren Position und Ausrichtung (sechs Freiheitsgrade bzw. Dimensionen) getrackt werden kann. Außerdem ist es möglich, Positionen und Richtungen von Ebenen in fester Relation zu dem Pin zu definieren. Eine erste Ebene könnte theoretisch senkrecht zur symmetrischen Pinachse positioniert sein. Eine weitere Ebene könnte koaxial zur Achse und senkrecht zur ersten Ebene positioniert sein. Der Ausrichtungs- bzw. der Aufnahmemechanismus für den Pin am Beckenregistrierungsrahmens ist in einer solchen Art und Weise konstruiert, dass der Pin nur in einer definierten Ausrichtung zu den vier vorher genannten anatomischen Landmarken, also den ASIS- oder Pubis-Punkten angeordnet werden kann. Damit ist der Pin immer in definierter Weise zur anatomischen vorderen Beckenebene ausgerichtet, und er zeigt die Richtung und die Position der vorderen Beckenebene und der mittleren Sagittalebene an. Natürlich könnte der Pin auch noch in analoger Weise an unterschiedlichen Positionen am Rahmen und in definierter Relation zu anatomischen Becken-Landmarken angeordnet werden, um andere Ebenenausrichtungen oder Achsenausrichtungen zu bestimmen.

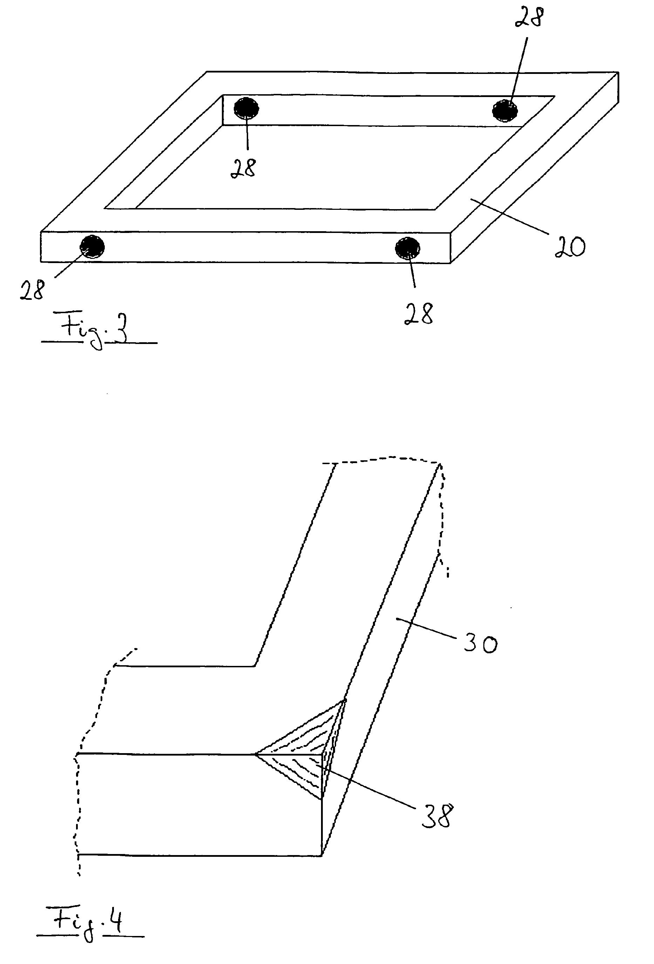

Wie oben schon angedeutet wurde, kann ein Becken-Registrierungsrahmen wie zum Beispiel derjenige, der in Figur 1 mit dem Bezugszeichen 1 aufgezeigt wird, auch durch eine Fluoroskopie-Registrierung räumlich bestimmt werden. In der Figur 3 ist nur schematisch ein Rahmen 20 angedeutet, der vier Röntgenmarker 28 trägt, die an unterschiedlichen Stellen an ihm befestigt sind. Es ist nun möglich, eine automatische Erfassung dieser röntgenpositiven Marker 28 in einem für Röntgenstrahlung durchlässigen Rahmen 20 durchzuführen, wenn die Marker an festen Positionen in Bezug auf die Beckenlandmarken am Rahmen angebracht werden. Wenn der Rahmen am Patientenbecken angesetzt wird, wie es oben beschrieben wurde, haben die Marker 28 eine feste Position gegenüber dem Becken und gegenüber den Beckenebenen. Die Konstruktion und die Abmessungen des Rahmens und die Positionen der Marker sind in einem Navigations-Computerprogramm implementiert. Die Anzahl der Marker ist nicht fest definiert. Es kann z.B. auch nur ein einziger Marker vorhanden sein, der dementsprechend konstruiert ist und durch seine Form die Lage im Röntgen- oder CT-Bild erkennbar macht.

Bei einer Ausführungsform werden der Becken-Registrierungsrahmen 20 und ein Beckensensor (optische Referenzanordnung oder elektromagnetischer Sensor) am Patientenbecken angesetzt, bevor Röntgenbilder bzw. Fluoroskopiebilder erstellt werden. Wenn ein C-Bogen-Fluoroskopiegerät verwendet wird, kann ein Kalibrierungs-Kit am C-Bogen angebracht werden. Nach dem Erstellen der Bilder mit dem am Patienten angesetzten Becken-Registrierungsrahmen ermittelt das Navigations-Computerprogramm automatisch die Position der Marker, während der Rahmen aufgrund seiner Strahlendurchlässigkeit nicht sichtbar ist. Die Position der Marker bzw. des Rahmens wird in Relation zum Beckensensor bzw. zur Beckenreferenzanordnung gespeichert. Somit kann das System die Richtungen der anatomischen Beckenebenen (vordere Beckenebene, mittlere Sagittalebene) in Relation zum Beckensensor bzw. zur Referenzanordnung ermitteln.

Es besteht im Rahmen der Erfindung weiterhin die Möglichkeit, die Röntgenmarker nicht als separate Marker dem Rahmen hinzuzufügen, sondern als Teile des Rahmens auszugestalten. In Figur 4 ist aufgezeigt, wie eine Ecke eines Rahmens 30 durch ein röntgenpositives Material ersetzt wird, das dann einen Röntgenmarker 38 bildet. Die Positionsermittlung für die Ebenen würde dann mit mehreren solcher Röntgenmarker 38 ebenso erfolgen wie oben für die separaten Marker 28 beschrieben. Zusätzlich oder auch allein stehend könnte eine Kantenerfassung für den Rahmen durchgeführt werden. Wenn die Kanten in definierter und fester Relation zu den Beckenkontaktpunkten stehen kann die Software einen Kantenerfassungsallgorithmus verwenden, um die Position des Rahmens zu bestimmen. Dazu müssen natürlich zumindest die Kanten des Rahmens für Röntgenstrahlen undurchlässig sein.

Es soll noch einmal bemerkt werden, dass die in den Figuren 3 und 4 gezeigten Rahmen 20, 30 nur schematische Darstellungen sind. Natürlich kann die dort aufgezeigte Anbringung von Röntgenmarkern auf einen Rahmen übertragen werden, wie er beispielsweise in Figur 1 dargestellt ist.

Die erfindungsgemäße Beckenregistrierung mittels einer Fluoroskopie-Registrierung des Becken-Registrierungsrahmens kann wie folgt ablaufen: Zunächst wird der Rahmen an das Becken angesetzt (wie in Figur 1 gezeigt ist) und dann wird ein Beckensensor (optischer oder elektromagnetischer Sensor für ein entsprechendes Trackingsystem) am Becken abgebracht. Hierauf folgt die Erstellung der Fluoroskopiebilder, wobei ein Fluoroskopie-Registrierungskit verwendet wird, und die Position des Beckensensors wird gespeichert. Danach wird durch die Navigationssoftware eine automatische Erfassung der Rahmenmarker in den Fluoroskopie- bzw. Röntgenbildern vorgenommen, und die Position der Marker bzw. des Rahmens kann in Relation zu dem Beckensensor errechnet werden.

Mit den oben erhaltenen Information ist es möglich, die Richtung der anatomischen Beckenebenen, die aus der Ausrichtung des Rahmens ermittelt werden, in einem Koordinatensystem aufzuzeigen, dass durch den Beckensensor definiert wird.

Optional können noch weitere Schritte erfolgen, nämlich beispielsweise die Akquirierung von Becken-Landmarkenpunkte (z.B. ASIS- oder Pubis-Punkten) und zwar mit einer Pointer-Vorrichtung oder durch automatische Detektion in den erhaltenen Bildern. Ferner kann die Position der errechneten Beckenebenen in Relation zu einer akquirierten Beckenlandmarke ermittelt und dargestellt werden, und schließlich ist es noch optional möglich, über eine Bildanzeigevorrichtung die Beckenebenen virtuell in Relation zu vorab aufgenommenen Bilddaten darzustellen.

## Patentansprüche

1. Vorrichtung zur Registrierung der Position bzw. der Ausrichtung eines Patientenbeckens für die medizintechnische Navigation, wobei die Vorrichtung einen Becken-Registrierungsrahmen (1, 20, 30) umfasst, der Positionierungselemente (2) trägt, die an definierten Beckenpunkten ansetzbar sind, wobei der Vorrichtung, insbesondere dem Rahmen (1, 20, 30), mindestens ein Positionsgeber (7, 8, 28, 38) zugeordnet ist, der in eindeutiger definierbarer Position und/oder Ausrichtung gegenüber dem Rahmen angeordnet ist oder angeordnet werden kann, **dadurch gekennzeichnet, dass** der Positionsgeber eine Magnettrackingspule (7, 8) ist, deren Position und/oder Ausrichtung von einem medizintechnischen Magnettrackingsystem erfassbar ist und der Rahmen (1) mindestens eine Positionierungs- und/oder Ausrichtungshilfe, insbesondere eine Aufnahme (5), für einen frei handhabbaren Sensorträger (6) aufweist, der eine darin ortsdefiniert angebrachte Magnettrackingspule (7) trägt, wobei die Positionierungs- und/oder Ausrichtungshilfe bzw. Aufnahme (5) den Sensorträger richtungs- und/oder positionsbestimmend in definierter Position gegen den Rahmen anordnet.

## Claims

1. A device for registering the position and/or the orientation of a patient's pelvis for medical navigation, wherein the device comprises a pelvic registering frame (1, 20, 30) bearing positioning elements (2) which can be placed at defined pelvic points, wherein the device, in particular the frame (1, 20, 30), is assigned at least one position transmitter (7, 8, 28, 38) which is or can be arranged in an unambiguously definable position and/or orientation relative to the frame, **characterised in that** the position transmitter is a magnetic tracking coil (7, 8), the position and/or orientation of which can be detected by a medical magnetic tracking system, and the frame (1) comprises at least one positioning and/or orientation aid, in particular a recess (5), for a freely manageable sensor carrier (6) which bears a magnetic tracking coil (7) which is attached in a defined location in it, wherein the positioning and/or orientation aid or recess (5) arranges the sensor carrier in a defined position relative to the frame, so as to determine its direction and/or position.

## Revendications

1. Dispositif pour enregistrer la position ou l'orientation d'un bassin de patient pour la navigation médicale, le dispositif comportant un cadre de repérage de bassin (1, 20, 30) portant des éléments de positionnement (2) qui peuvent être placés sur des points définis du bassin, dans lequel au moins un transmetteur de position (7, 8, 28, 38) est associé au dispositif, en particulier au cadre (1, 20, 30), lequel transmetteur de position est agencé ou peut être agencé dans une position et/ou une orientation définissable de manière univoque par rapport au cadre, **caractérisé en ce que** le transmetteur de position est une bobine de suivi magnétique (7, 8) dont la position et/ou l'orientation peut être détectée par un système de suivi magnétique médical et le cadre (1) comporte au moins une aide au positionnement et/ou à l'orientation, en particulier un récepteur (5), pour un support de capteur librement manipulable (6), lequel support porte une bobine de suivi magnétique (7) fixée à un endroit défini dans celui-ci, l'aide au positionnement et/ou à l'orientation ou le récepteur (5) place le support de capteur de manière déterminée en termes d'orientation et/ou de position dans une position définie par rapport au cadre.
